(19) Europäisches Patentamt

European Patent Office

Office européen des brevets

(11) **EP 4 487 801 A1**

(12) **EUROPEAN PATENT APPLICATION**

(43) Date of publication:
08.01.2025 Bulletin 2025/02

(21) Application number: 23183898.8

(22) Date of filing: **06.07.2023**

(51) International Patent Classification (IPC):
*A61B 34/00* (2016.01)    *A61K 9/00* (2006.01)
*A61K 9/48* (2006.01)    *A61K 9/50* (2006.01)

(52) Cooperative Patent Classification (CPC):
**A61B 34/72; A61K 9/0009; A61K 9/4808;**
**A61K 9/4891;** A61B 2017/00345

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Max-Planck-Gesellschaft zur Förderung der Wissenschaften e.V.**
**80539 München (DE)**

(72) Inventors:
- **Sitti, Metin**
**80539 München (DE)**
- **Wrede, Paul**
**80539 München (DE)**
- **Aghakhani, Amirreza**
**Newcastle upon Tyne NE1 7RU (GB)**

(74) Representative: **Richardt Patentanwälte PartG mbB**
**Wilhelmstraße 7**
**65185 Wiesbaden (DE)**

(54) **ACOUSTIC TRAPPING OF PARTICLES**

(57) Disclosed is an acoustic trapping assembly (100) comprising a focused ultrasound transducer (102) and a plurality of hollow microrobots (110). The focused ultrasound transducer (102) is configured for generating an acoustic pressure maximum within a focal area (106) of a focused ultrasound beam (104) emitted by the focused ultrasound transducer (102) with the plurality of hollow microrobots (110) being acoustically trapped within the acoustic pressure maximum under fluid flow in a fluidic medium (120). The hollow microrobots (110) comprise solid shells (112) filled with gas.

Fig. 1

## Description

## FIELD OF THE INVENTION

The invention relates to the field of acoustic trapping. More particularly, the invention relates to an acoustic trapping assembly.

## BACKGROUND

For an external manipulation of a fluidic system with a fluidic medium, e.g., acoustic trapping assemblies using ultrasound for acoustic trapping and manipulation of microbubbles of gas are used. However, using gas bubbles may have different drawback, like dissolution of the bubbles, i.e., a shrinking of bubble size, fragmentation of the bubbles, i.e., a breaking-up into smaller bubbles, and/or coalescence of the bubbles, i.e., a merging of smaller bubbles close together. Therefore, there is a need for an acoustic trapping assembly able to avoid such problems.

It is an objective to provide for an acoustic trapping assembly. The objectives underlying the invention are solved by the features of the independent claims.

## SUMMARY

In one aspect an acoustic trapping assembly is disclosed comprising a focused ultrasound transducer and a plurality of hollow microrobots. The focused ultrasound transducer is configured for generating an acoustic pressure maximum within a focal area of a focused ultrasound beam emitted by the focused ultrasound transducer with the plurality of hollow microrobots being acoustically trapped within the acoustic pressure maximum under fluid flow in a fluidic medium. The hollow microrobots comprise solid shells filled with gas. For example, the focused ultrasound transducer is a single-lens focused ultrasound transducer.

Examples may implement an acoustic trapping of hollow microrobots under fluid flow using a focused ultrasound transducer, e.g., a single-lens focused ultrasound transducer. Examples may enable an efficient and precise manipulation of the trapped hollow microrobots. The hollow microrobots may be robustly trapped within the focal area of the focused ultrasound (FUS) beam, i.e., the focused ultrasound comprised by the beam, which is emitted by the focused ultrasound transducer, e.g., a single-lens focused ultrasound transducer. For example, the acoustic trapping assembly may enable a trapping of the hollow microrobots with sub-millimeter accuracy.

The acoustic manipulation and control of trapped hollow microrobots open up a wide array of possibilities for applications in different fields such as physics, chemistry, biology, and medical research.

The trapped hollow microrobots may, e.g., be navigated inside confined, hard-to-access regions to reach a target region using the focused ultrasound waves as a wireless actuation measure. These confined regions may be regions within a fluid conducting element, e.g., a branching fluid conducting element, with a fluid flowing through the fluid conducting element.

The hollow microrobots of the acoustic trapping assembly trapped using a beam of focused ultrasound waves emitted, e.g., by a single-lens focused ultrasound transducer, may be held in place and/or moved by moving the respective focused ultrasound transducer under a wide variety of flow conditions. These hollow microrobots may combine the benefits of gas bubbles in liquid media with the material stability of synthetic microrobots.

Due to their solid walls, using hollow microrobots may avoid problems occurring with gas bubbles, like dissolution, i.e., a shrinking of bubble size, fragmentation, i.e., a breaking-up into smaller bubbles, and/or coalescence, i.e., a merging of smaller bubbles close together. Therefore, the hollow microrobots of the acoustic trapping assembly may be better controllable and more stable compared to gas bubbles.

Furthermore, the solid walls of the hollow microrobots may enable a precise control of position of the trapped hollow microrobots even in confined spaces and under fluid flow.

The hollow microrobots consist of a solid, e.g., spherical, shell and a gas-filled, e.g., air-filled, core making them stable and buoyant. They are also scalable and may be mass-produced. The presented technology possesses the advantageous attributes of low expense, miniature size, and user-friendly operation, making it amenable to integration with existing ultrasound systems. No complex fabrication of advanced focused ultrasound transducer setups with multiple transducers is required, but a single-lens focused ultrasound transducer may be sufficient for an effective trapping.

For example, a buoyancy of the hollow microrobots may be adjusted to match a fluidic medium they are immersed in. For example, the hollow microrobots may be adjusted such that their buoyance, i.e., a buoyancy force acting on the hollow microrobots in the fluidic medium equals or exceeds a gravitational force acting on the hollow microrobots. This may, e.g., be achieved by a selection of a gas being used for filling the hollow microrobots depending on its density, a selection of a material used for manufacturing the solid shells of the hollow microrobots depending on its density, an adjustment of a size and/or shell thickness of the hollow microrobots. Thus, issues of sedimentation may be avoided and a manipulation of the hollow microrobots using ultrasound acoustics may be made more manageable.

The medium, in with the hollow microrobots are trapped by the focused ultrasound transducer may, e.g., have a viscosity smaller than 30 mPa·s, smaller than 25 mPa·s, smaller than 20 mPa·s, smaller than 15 mPa·s, smaller than 10 mPa·s, smaller than 7.5 mPa·s, or smaller than 6 mPa·s. For example, the viscosity may be larger than 0.5 mPa·s. For example, the viscosity may be 4.5 mPa·s. For example, the viscosity may be 1 mPa·s.

Herein, a microrobot refers to a micro-scale particle with a solid shell filed with gas, i.e., a gas core. The microrobot may, e.g., have a spherical form. The solid shell may, e.g., be gas-tight.

The hollow microrobots may have a negative acoustophoretic contrast factor resulting in the trapping within the acoustic pressure maximum of the focal area of the focused ultrasound beam emitted by the single-lens focused ultrasound transducer.

The acoustophoretic contrast factor of the hollow microrobots may, e.g., be determined using the following equation assuming a one-dimensional (1D) standing wave and a non-viscous fluidic medium:

$$\phi = \frac{1}{3}\left(\frac{5\frac{\rho_p}{\rho_m} - 2}{2\frac{\rho_p}{\rho_m} + 1} - \frac{\kappa_p}{\kappa_m}\right)$$

Whereas $\rho_p$, $\rho_m$, $\kappa_p$ and $\kappa_m$ are a density of the hollow microrobots and medium as well as a compressibility of both the hollow microrobots and medium, respectively. According to this equation, the acoustophoretic contrast factor is mainly dependent on the material and geometrical properties of the hollow microrobots. Additionally, for core shell-particles like the hollow microrobots the mean density depends on the microrobots' inner and outer radius $r_i$ and $r_o$ as $\rho_p = \rho_{shell}(1-\alpha^3)$, where $\alpha = r_i/r_o$. For solid particles, this ratio would be 0, while for core shell particles like the hollow microrobots it may be between 1 and 0. The hollow microrobots may e.g., be hollow borosilicate microrobots having an $\alpha$ of about 0.98. The acoustophoretic contrast factor $\phi$ decreases with increasing $\alpha$. For example, around $\alpha = 0.9$ the acoustophoretic contrast factor $\phi$ may reach 0. When further increasing $\alpha$, the acoustophoretic contrast factor $\phi$ may get negative rapidly. Consequently, the hollow microrobots of the acoustic trapping assembly may have a highly negative acoustophoretic contrast factor $\phi$. If $\phi > 0$, particles may be attracted to pressure nodes of a 1D standing wave, but for $\phi < 0$ they may be attracted to the pressure anti-nodes, like the hollow microrobots of the acoustic trapping assembly described herein. It may be noted that the acoustophoretic contrast factor $\phi$ does not depend on a radius value alone, but rather on a ratio of inner to outer radius of the hollow microrobots.

Using the acoustophoretic contrast factor $\phi$ for the hollow microrobots, an acoustic radiation force acting on the hollow microrobots may be determined, assuming Gor'kov potential as:

$$F_R = 4\pi\phi k_x r_o^3 E_{ac} \sin(2k_x x)$$

With $k_x$, $E_{ac}$, and $x$ being wave number, acoustic energy density, and spatial position of the hollow microrobots. For this equation a negative acoustophoretic contrast factor $\phi$ results in a negative acoustic radiation force $F_R$ acting on the hollow microrobots. This may change behavior of the hollow microrobots inside the acoustic field. While solid spherical particles, which usually have positive $\phi$, are trapped inside a pressure node, the hollow microrobots with negative $\phi$ are trapped inside the pressure maximum. The hollow microrobots may be trapped directly in a maximum pressure region of the focal area of the focused ultrasound waves emitted by a single-lens focused ultrasound transducer. This may enable a precise control of these trapped hollow microrobots in all axes using only the single-lens focused ultrasound transducer. Thus, any need for utilization of complex acoustic actuation systems, e.g., including acoustic vortex tweezers, multiple ultrasonic actuators, or the like, may be avoided.

Examples enable a trapping of hollow microrobots against high flow speeds, e.g., up to 119.6 mm/s. For example, the acoustic trapping assembly may enable an effective trapping of the hollow microrobots even under physiologically relevant flow conditions.

For example, the acoustically trapped plurality of hollow microrobots form an aggregate. Forming an aggregate may have the beneficial effect, that an effect of the aggregated hollow microrobots may be combined, e.g., accumulated. Forming an aggregate, the plurality of trapped hollow microrobots may be manipulated, e.g., moved, together without losing the individual characteristics of the individual hollow microrobots.

At the focal area of the focused ultrasound waves, the hollow microrobots may be robustly trapped forming an aggregate. For example, supplying the hollow microrobots successively under a fluid flow through the focal area, they may aggregate and grow in size forming the stable aggregate, e.g., in a size of around 500 μm diameter, under the acoustic radiation force generated by a single-lens focused ultrasound transducer. They may, e.g., aggregate, while being stably separated from each other due to their solid shells, avoiding coalescence issues.

For example, the hollow microrobots are polydisperse regarding one or more of the following: form, size, shell thickness. For example, the hollow microrobots are monodisperse regarding one or more of the following: form, size, shell thickness. Depending on an individual application, an individual combination of, e.g., polydisperse or monodisperse, hollow microrobots may be used.

For example, the hollow microrobots have spherical forms. For example, the hollow microrobots have ellipsoidal forms.

For example, the hollow microrobots have a size within a range from 2.5 μm to 17.5 μm. For example, the hollow microrobots have a size within a range from 8 μm to 12 μm.

For example, a ratio of an inner radius to an outer radius of the hollow microrobots is at least 0.90. For example, the ratio of the inner radius to the outer radius of the hollow microrobots is at least 0.95. For example, the ratio of the inner radius to the outer radius of the hollow microrobots is at least 0.98. For a large ratio the acoustophoretic contrast factor becomes negative. Thus, the ratio may be selected to ensure a negative acoustophoretic contrast factor of the hollow microrobots.

For example, the solid shells of the hollow microrobots have a thickness within a range from 100 nm to 1 $\mu$m . For example, the solid shells of the hollow microrobots have a thickness within a range from 250 nm to 750 nm. For example, the solid shells of the hollow microrobots have a thickness within a range from 400 nm to 600 nm.

For example, the solid shells of the hollow microrobots are gas-tight. Examples may ensure that the gas is maintained within the solid shells of the hollow microrobots.

For example, the solid shells of the hollow microrobots comprise at least one layer made from borosillicate. Using borosillicate may provide a solid, gas-tight shell with a suitable density.

For example, the solid shells of the hollow microrobots comprise a coating. The coating may be used to add functionalities to the hollow microrobots preparing them for specific tasks. For example, the coating of the solid shells of the hollow microrobots comprises a reagent. Thus, the hollow microrobots may, e.g., be used as carriers for carrying the reagent to a target position, e.g., by moving the trapped hollow microrobots to the target position.

There may be a wide range of penitential applications for the acoustic trapping assembly. For example, the hollow microrobots may be usable targeted drug delivery in an animal or a human body. The hollow microrobots being trapped by the single-lens focused ultrasound transducer may be prevented from being washed away. Thus, e.g., when used for delivering a drug, the hollow microrobots may be prevented from being washed away and, e.g., from unwanted side effects. Using the acoustic trapping, e.g., a stable aggregate may be formed and manipulated, even in presence challenging flow conditions.

In view of physiologically relevant flow conditions, e.g., high-velocity flow rates as they may occur in a human circulatory system, microparticle like the hollow microrobots of the acoustic trapping assembly may be advantageous compared to nanoparticles. For example, a margination and effective delivery of particle-based drugs to a target disease area may be limited by flow conditions. Physiological flow characteristics of blood vessels may cause less than 1% of a plurality of supplied nanoparticles to reach a targeted disease area. This problem may become even worse in the case of chemotherapeutic drugs, where a large dose may have to be administered to compensate for a low accumulation of nanoparticles, which may cause undesired side effects. In comparison, microparticles may be better suited for margination to the vascular wall and may act as carriers, e.g., for nanoparticle drugs. However, even though tuning size and modulus of passive microparticles may increase their margination efficiency, their local accumulation at a desired cellular region is not actively controllable. Therefore, using actively controlled microparticles, like the hollow microrobots of the acoustic trapping assembly, may in addition to the benefits of microparticles enable an active control of accumulation at a desired target.

The trapped hollow microrobots may, e.g., be navigable inside confined, hard-to-access regions to reach the targeted disease region using the focused ultrasound waves as a wireless actuation measure. Using surface chemistry methods, the microrobots' bodies may, e.g., be coated, i.e., decorated, with a therapeutic payload, e.g., gene or drug molecules. Upon reaching the target region, e.g., tissue, the hollow microrobots may release the payload, e.g., either passively through diffusion or actively by external stimuli, like ultrasound.

The proposed acoustic trapping assembly may have the potential to significantly impact, e.g., the field of targeted drug delivery. As ultrasound agents, the hollow microrobots may not only provide excellent contrast for deep tissue imaging, but they may further form stable aggregates at specific target locations and be manipulated within a circulatory system while under flow. Furthermore, like other solid microparticles, the hollow microrobots may have the advantage of high-throughput fabrication and versatile chemical modification capabilities, such as surface coating, drug loading, or cargo attachment. Such drug-loaded hollow microrobots trapped with sub-millimeter accuracy, e.g., in proximity to diseased tissue may be used for a targeted release of drugs, e.g., passively through diffusion or actively through a series of ultrasound pulses.

For example, the focused ultrasound beam emitted by the focused ultrasound transducer has a frequency within a range from 250 kHz to 5 MHz. For example, the focused ultrasound beam emitted by the focused ultrasound transducer has a frequency within a range from 500 kHz to 3.5 MHz.

For moving the trapped hollow microrobots trough confined regions, e.g., of a fluid conducting element, the focused ultrasound transducer may be moved along a trajectory navigating the trapped hollow microrobots along the same trajectory. For example, a computer device may be used for controlling a moving unit, like a robotic arm, configured for moving the focused ultrasound transducer along the trajectory. Thus, the trapped hollow microrobots may, e.g., be navigated inside confined, hard-to-access regions, e.g., to reach a target region, using the focused ultrasound waves as a wireless actuation measure.

It is understood that one or more of the aforementioned embodiments may be combined as long as the combined embodiments are not mutually exclusive.

## BRIEF DESCRIPTION OF THE DRAWINGS

In the following, examples are described in greater detail making reference to the drawings in which:

Fig. 1 shows an exemplary acoustic trapping assembly;

Fig. 2 shows an exemplary application of the exemplary acoustic trapping assembly of Fig. 1;

Fig. 3 shows a schematic representation of an exemplary hollow microrobot;

Fig. 4 shows a light microscope image of a plurality of polydisperse hollow microrobots and an SEM image of a single hollow microrobot;

Fig. 5 shows an exemplary graph of the acoustophoretic contrast factor of the hollow microrobots;

Fig. 6 shows an exemplary representation of a dependency of the acoustic radiation force on an outer radius and a ratio of inner to outer radius of the hollow microrobots;

Fig. 7 shows an exemplary finite element simulation of an acoustic pressure distribution around a hollow microrobot immersed into a standing acoustic wave;

Fig. 8 shows an exemplary finite element simulation of a pressure applied in a tubing;

Fig. 9 shows an exemplary experimental setup comprising an acoustic trapping assembly;

Fig. 10 shows exemplary microscope images depicting hollow microrobots flowing through a tubing;

Fig. 11 shows exemplary time history images of a pixel intensity;

Fig. 12 shows exemplary normalized concentrations of microrobots within a trapping region over time;

Fig. 13 shows an exemplary control and retrieval of hollow microrobots against a fluid flow;

Fig. 14 shows time laps illustrating a retrieval of hollow microrobots;

Fig. 15 shows an exemplary movement of acoustically trapped hollow microrobots;

Fig. 16 shows an exemplary 2D movement of trapped hollow microrobots;

Fig. 17 shows exemplary ultrasonographic images of a needle positioning and a subsequent hollow microrobots injection;

Fig. 18 shows a schematic representation of a functionalization method for binding an anti-HER2 antibody to a hollow borosilicate microrobot;

Fig. 19 shows an exemplary movement of hollow microrobots inside a bifurcated microfluidic channel chip;

Fig. 20 shows exemplary microscope images depicting a trapping and subsequent movement of hollow microrobots; and

Fig. 21 shows exemplary light microscope images of SKBR3 cells embedded into a cell culture chip.

## DETAILED DESCRIPTION

In the following, similar elements are denoted by the same reference numerals.

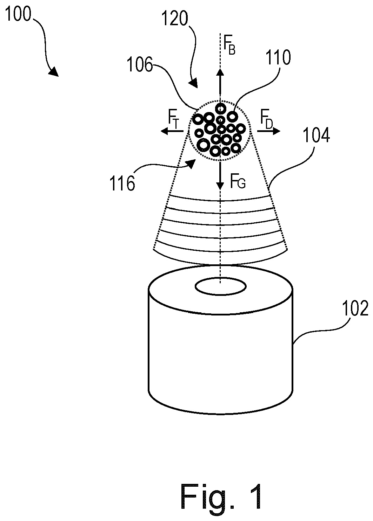

Fig. 1 shows an exemplary acoustic trapping assembly 100. The acoustic trapping assembly 100 comprises a single-lens focused ultrasound transducer 102 and a plurality of hollow microrobots 110. The single-lens focused ultrasound transducer 102 is configured to emit a focused ultrasound beam 104. Within a focal area 106 of a focused ultrasound beam 104 an acoustic pressure maximum is generated. The plurality of hollow microrobots 110 is acoustically trapped within the acoustic pressure maximum. The hollow microrobots 110 comprise solid shells filled with gas. Even in a fluidic medium 120 under fluid flow, the hollow microrobots 110 may be efficiently trapped in place and form an aggregate 116.

The hollow microrobots 110 may be trapped, e.g., inside a tubing, against a flow induced drag force $F_D$ acting on them. The focused ultrasound beam 104 used to trap the hollow microrobots 110 may, e.g., be arranged below, above, or lateral of the hollow microrobots 110. Here, below refers to the direction, in which a gravitational force $F_G$ acts, above to the direction in which a buoyancy force $F_B$ acts, and lateral in a direction perpendicular to the directions, in which the gravitational force $F_G$ and buoyancy force $F_B$ act. The emitted focused traveling ultrasound waves of beam 104 apply a trapping force $F_T$ to the hollow microrobot 110 counter acting $F_D$. The trapping force $F_T$ results from an acoustic radiation force $F_R$ generated by the focused ultrasound beam 104. Additionally, the gravitational force $F_G$ as well as the buoyancy force $F_B$ arising from the hollow nature of the microrobots 110 are acting on the microrobots 110.

The acoustic trapping assembly 100 illustrated in Fig. 1 enables a stable trapping of the hollow microrobots 110 taking advantage of their hollow nature. A stable trapping may even be achieved under fluid flow using the focused ultrasound beam 104 emitted by the single-lens focused ultrasound transducer 102. Being trapped within the focal area 106 of the focused ultrasound beam 104, the hollow microrobots 100 may further be manipulated, e.g., move with the fluid flow, against the fluid flow and/or perpendicular to the fluid flow, e.g., by moving the focal area 106, i.e., moving the focused ultrasound transducer 102.

The hollow microrobots 110, illustrated in Fig. 1, comprise a solid spherical shell and a gas-filled core, making them stable and buoyant. Thus, the hollow microrobots 110 may be enabled to implement a combination of properties of gas bubbles and solid shell materials of synthetic microrobots. Therefore, the hollow microrobots 110 may be suitable for effective margination in confined environments, scalable and mass-producible. This may enable a usage of large numbers of hollow microrobots 110, e.g., more than $10^6$ microrobots/mL, being supplied to the fluid medium.

The trapped hollow microrobots 110, may be successively supplied and trapped resulting in a growth in size forming a stable aggregate under the acoustic radiation force. The size of such an aggregate may, e.g., be around 500 $\mu$m diameter, while the individual trapped hollow microrobots 110 are still separated from each other. This separation due to the solid shells may eliminate coalescence issue as in case of gas-filled microbubbles. Thus, besides trapping capabilities, the acoustic trapping assembly 100 may further enable a manipulation, e.g., moving, of the aggregate within the fluid medium 120, e.g., within a microchannel, by steering the single-lens focused ultrasound transducer 102.

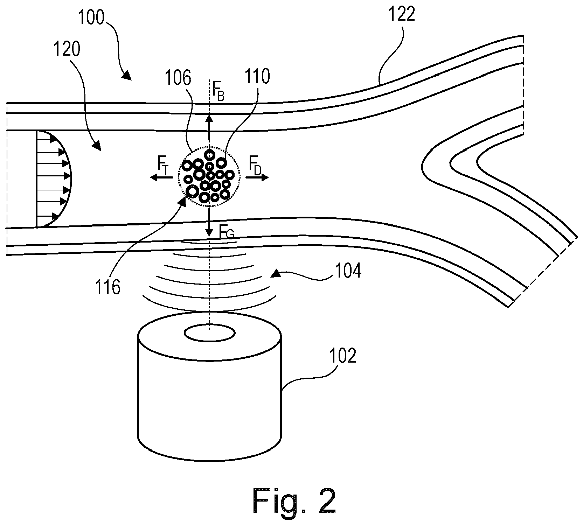

Fig. 2 shows an exemplary application of the exemplary acoustic trapping assembly 100 of Fig. 1 with the hollow microrobots 110 trapped in a bifurcated pipe or tubing 122 against the flow induced drag force $F_D$ resulting from a liquid medium 120 streaming through the tubing 122. As illustrated in Fig. 2, the hollow microrobots 110 may be robustly trapped within the focal area 106 of the focused ultrasound beam 104 inside a pipe 122 even under high fluid flows. For example, a large number of hollow microrobots 110, e.g., more than $10^6$ microrobots/mL, may be injected into a body.

Also, in case of a confined space, like a pipe 122, an aggregate, e.g., with a diameter of about 500 $\mu$m, may be formed, while the individual trapped hollow microrobots 110 are still separated from each other. This separation due to the solid shells may eliminate coalescence issue as in case of gas-filled microbubbles. Besides trapping capabilities, the acoustic trapping assembly 100 may further enable a manipulation, e.g., moving, of the aggregate within the fluid medium 120, e.g., within a pipe 122, by steering the single-lens focused ultrasound transducer 102.

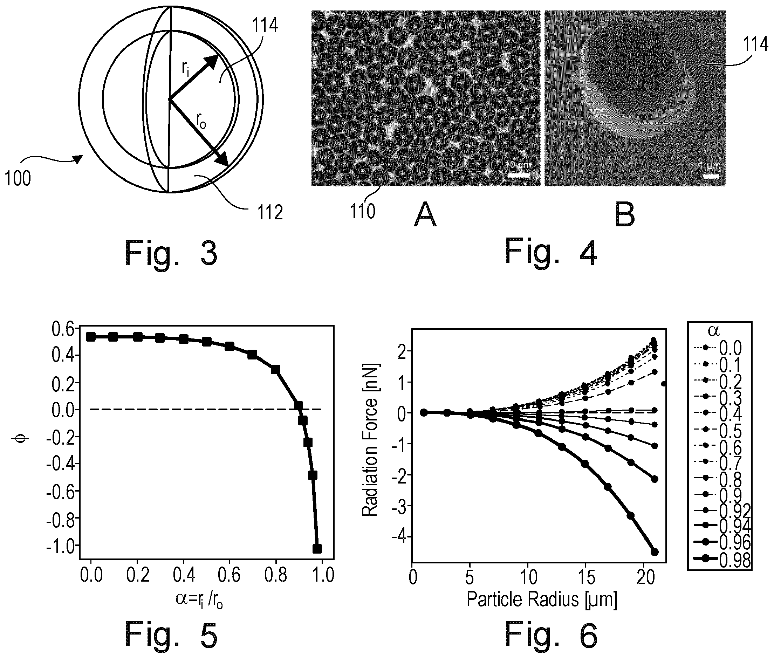

Fig. 3 shows a schematic representation of an exemplary hollow microrobot 110 comprising a solid shell 112 filled with a gas-filled core 114. The solid shell 112 may, e.g., be made from borosilicate. The core 114 may, e.g., be filled with air. Thus, the hollow microrobot 110 is provided in form of a hollow core-shell microparticle, i.e.,

a microparticle comprising a solid shell 112 surrounding a hollow core 114. The shell 112 has an inner radius $r_i$ and an outer radius $r_o$. The ratio $\alpha$ of the inner radius $r_i$ to an outer radius $r_o$ of the hollow microrobot 110 is $\alpha = r_i/r_o$.

Fig. 4 shows a light microscope image of a plurality of polydisperse hollow microrobots 110 (left image A) and a scanning electron microscopy (SEM) image (right image B) of a single hollow microrobot 110. As shown in the left image A of Fig. 4, upon immersion of the dry hollow microrobots 110 inside a fluidic medium, the gas-filled core 114 of the hollow microrobots 110 may become clearly visible under a microscope as a dark-shaded area due to a light contrast at a fluid-solid-gas interface. These hollow microrobots 110 shown are, e.g., polydisperse with a size range from 2.5 to 17.5 $\mu$m. A sample measurement of a few hundred hollow microrobots 110 revealed that most of the hollow microrobots 110 may, e.g., have a larger size distribution, i.e., a size within a range of size from 8 to12 $\mu$m. A shell thickness of a hollow microrobot 110 may be determined to be around 500 nm, as shown in the SEM-analysis depicted in right image B of Fig. 4.

For the SEM imaging, the hollow borosilicate microrobot 110 were coated with 10 nm Au in preparation. The Au served as a conductive surface to decrease charging during the imaging procedure. After the acquisition of SEM images of intact hollow microrobot 110, the borosilicate shell 112 of the hollow microrobot 110 was cracked as shown in the right image B of Fig. 4. For this purpose, a glass slide was positioned parallel to the sample, and pressure was applied manually. The cracked hollow microrobots 110 were imaged subsequently to determine the shell thickness of the hollow borosilicate hollow microrobots 110.

Fig. 5 shows an exemplary graph of the acoustophoretic contrast factor $\phi$ of the hollow microrobots for different ratios $\alpha$ of $r_i$ and $r_o$ of the hollow microrobots. For small $\alpha$, as in case of a solid particle, the acoustophoretic contrast factor $\phi$ is positive. When increasing the ratio $\alpha$, the acoustophoretic contrast factor $\phi$ gets negative inverting an acoustophoretic behavior of the hollow microrobots compared to solid particles. All values used for plotting the graph have been obtained by numerical simulations.

The acoustophoretic contrast factor of the hollow microrobots may, e.g., be determined using the following equation assuming a 1D standing wave and a non-viscous fluidic medium:

$$\phi = \frac{1}{3}\left(\frac{5\frac{\rho_p}{\rho_m} - 2}{2\frac{\rho_p}{\rho_m} + 1} - \frac{\kappa_p}{\kappa_m}\right)$$

Whereas $\rho_p$, $\rho_m$, $\kappa_p$ and $\kappa_m$ are a density of the hollow microrobots and medium as well as a compressibility of both the hollow microrobots and medium, respectively. According to this equation, the acoustophoretic contrast factor is mainly dependent on the material and geometrical properties of the hollow microrobots. Additionally, for core shell-particles like the hollow microbots the mean density depends on the microrobots' inner and outer radius $r_i$ and $r_o$ as $\rho_p = \rho_{shell}(1 - \alpha^3)$, where $\alpha = r_i/r_o$. For solid particles, this ratio would be 0, while for core shell particles like the hollow microrobots it may be between 1 and 0. The hollow microrobots may e.g., be hollow borosilicate microrobots have an $\alpha$ of about 0.98. As shown in Fig. 5, the acoustophoretic contrast factor $\phi$ decreases with increasing $\alpha$. Starting from, e.g., $\alpha = 0.9$, the acoustophoretic contrast factor $\phi$ may get 0. When further increasing $\alpha$, the acoustophoretic contrast factor $\phi$ may get negative rapidly. Consequently, the hollow microrobots of the acoustic trapping assembly may have a highly negative acoustophoretic contrast factor. If $\phi > 0$, particles may be attracted to pressure nodes of a 1D standing wave, but for $\phi < 0$ hollow microrobots may be attracted to the pressure anti-nodes, like the hollow microrobots of the acoustic trapping assembly. It may be noted that the contrast factor does not depend on radius value alone, but rather on a ratio of inner to outer radius of the hollow microrobots.

Fig. 6 shows an exemplary representation of a dependency of the acoustic radiation force on an outer radius of the hollow microrobots and the ratio $\alpha$. The graphs show that a radiation force acting on solid particles is positive, while the radiation force becomes negative for hollow particles. All values used for plotting the graphs have been obtained by numerical simulations.

Using the acoustophoretic contrast factor $\phi$ for the hollow microrobots, an acoustic radiation force acting on the hollow microrobots may be determined, assuming Gor'kov potential as:

$$F_R = 4\pi\phi k_x r_o^3 E_{ac} \sin(2k_x x)$$

With $k_x$, $E_{ac}$, and $x$ being wave number, acoustic energy density, and spatial position of the hollow microrobots. For this equation a negative acoustophoretic contrast factor $\phi$ results in a negative acoustic radiation force $F_R$ acting on the hollow microrobots of Fig. 6. This may change behavior of the hollow microrobots inside the acoustic field. While solid spherical particles, which usually have positive $\phi$, are trapped inside a pressure node, the hollow microrobots with negative $\phi$ are trapped inside the pressure maximum. The hollow microrobots may be trapped directly in a maximum pressure region of the focal area of the focused ultrasound waves emitted by the single-lens focused ultrasound transducer.

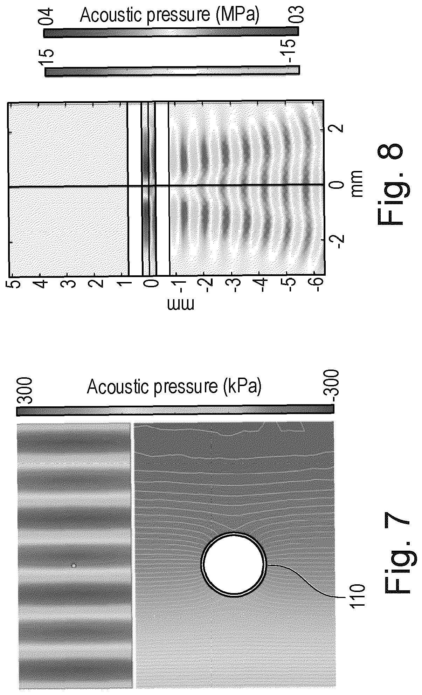

Fig. 7 shows an exemplary finite element simulation of an acoustic pressure distribution around a hollow microrobot 110 immersed into a standing acoustic wave. The finite element simulation is generated using COMSOL Multiphysics, a finite element analysis, solver, and simulation software package for various physics and engineering applications. Depicted is the acoustic pressure acting on a single hollow microrobot under a 2MHz planar ultrasound field.

Fig. 8 shows an exemplary finite element simulation of a pressure applied by a 2 MHz focused single-lens ultrasound transducer into a 500 $\mu$m tubing made from Tygon®, i.e., a flexible polymer tubing. Considering the simulation shown in Fig. 8, the hollow microrobots will be trapped directly in the maximum pressure regions of the focused ultrasonic focal area. This may enable the precise control of the trapped the hollow microrobots by controlling the focused ultrasonic focal area, i.e., the position of the focused ultrasonic focal area. The trapped hollow microrobots may, e.g., be controlled in all axes, i.e., in all three spatial directions, using only a single-lens focused ultrasound transducer. Thereby the need for utilizing any complex acoustic actuation system including, e.g., acoustic vortex tweezers and/or multiple ultrasonic actuators may be removed.

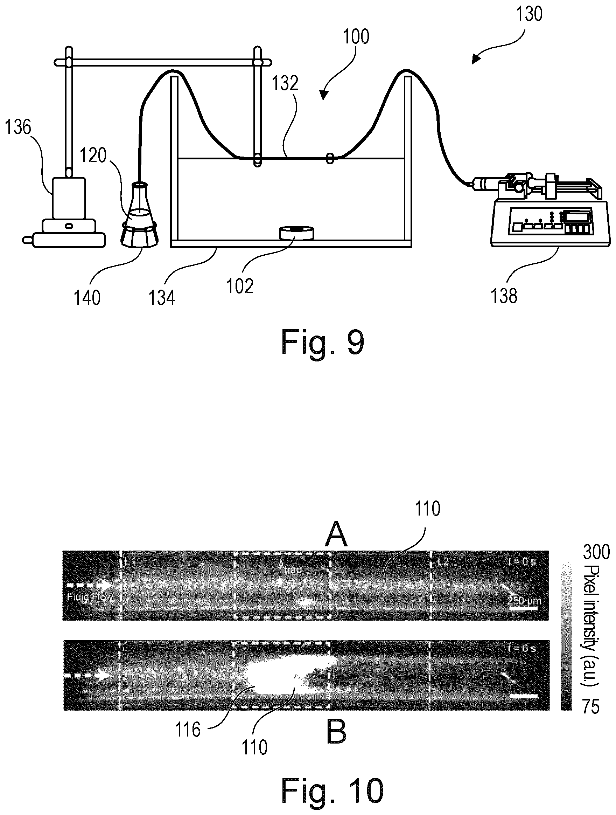

Fig. 9 shows an exemplary experimental setup 130 comprising an acoustic trapping assembly 100 for analyzing trapping dynamics of hollow microrobots of the acoustic trapping assembly 100 within a focal area of a focused ultrasound field generated using a single-lens focused ultrasound transducer 102. The experimental setup 130 schematically depicted in Fig. 9 may be used to evaluate the trapping efficacy of an acoustic travelling wave-induced agglomeration of hollow microrobots inside a 500 $\mu$m Tygon® tubing 132. A single-lens focused ultrasound transducer 102 with a central frequency of 2 MHz is arranged in a water tank 134. The tubing 132 is positioned under water using a manual x-y-z stage 136, while being in fluid connection with a syringe pump 138. The hollow microrobots are flushed with a fluid medium into the tubing 132 using the syringe pump 138. The liquid medium was stored in a fluid residue reservoir 140.

The hollow microrobots may, e.g., be injected into the 500 $\mu$m diameter Tygon® tubing 132 at different concentrations of 2.5, 5, and 10 mg/mL using the syringe pump 138. The focal area of the 2 MHz single-lens focused ultrasound transducer 102 is arranged within the tubing 132. In order to characterize the trapping performances of hollow microrobots within the acoustic trapping assembly 110 different flow rates, e.g., as listed above, and different acoustic pressure amplitudes may be applied. To determine the trapping behavior, the tests may be recorded by a high-speed camera (M310; Phantom, Inc), e.g., at 200 or 400 frames per second (fps). The resulting videos may, e.g., be subsequently analyzed using a custom Python code.

The experimental setup 130 may, e.g., be implemented using a single-lens focused ultrasound transducer 102 with a central frequency of 2 MHz (e.g., SU-101, Sonic Concepts, U.S.A.) arranged in a customized water tank 134, e.g., made from glass with dimensions of 30 x 30 x 20 cm. The single-lens focused ultrasound transducer 102 may be fixed to a bottom of the tank 134, e.g., using double-sided adhesive, and connected to an amplifier. A function generator (e.g., AFG 1022, Tektronix GmbH, Germany) attached to the amplifier may be used for generating an input for the amplifier including an input voltage and a frequency. To measure the output signal of the amplifier additionally an oscilloscope (e.g., MDO 4042C, Tektronix GmbH, Germany) may be connected to it. To determine the ability of the focused ultrasound trapping a Tygon® tubings 132 (Antylia Scientific, U.S.A.), e.g., with diameters of 500 $\mu$m or 4.7 mm, may be used. For precise positioning the tubing may be attached to a customized holder connected to an x-y-z stage 136. The hollow microrobots may be injected into the tubing 132 using a 24 G needle and a 1 ml volume syringe.

Besides geometric parameters of the hollow microrobots, characteristics of the surrounding acoustic field may influence the hollow microrobots' behavior. An acoustic field applied inside a tubing 132, e.g., made from Tygon®, used in this setup 130 may be measured using a hydrophone setup. The hydrophone may be inserted into the tubing 132 to measure the applied pressure in 3D around the acoustic focal area. The pressure may, e.g., be applied using a 500 kHz and 2MHz single-lens focused ultrasound transducers, e.g., with a 63.2 mm and 55 mm radius of curvature, positioned on the bottom of the tank 134. The tubing 132 is positioned inside the transducer focus using the manual x-y-z stage 136. A needle hydrophone may be used to measure the acoustic pressure amplitude at the focal area of the focused ultrasound waves. The hydrophone may be placed over the center of the transducer, while only the tip of the hydrophone is in contact with the water. The hydrophone may be connected to an oscilloscope to record the acquired signal. A burst of focused ultra sound may be sent with the transducer and the signal may be recorded using the hydrophone. This procedure may be repeated for several x-y positions. An acquired pressure map for different amplitude and frequency data at each specific point may be used to extrapolate for other locations. Finally, the pressure at the focal area of the transducer may also be measured, e.g., for different input voltages. Using the calibration sheet of the hydrophone, the measured voltage may be transformed into pressure. For 150 to 200 Vpp input voltage, maximum pressure amplitudes of 150-200 kPa were recorded at the focal area.

The results may be confirmed using finite element simulation performed in COMSOL Multiphysics. The results may prove that the focus area is characterized by a high positive pressure region surrounded by high negative pressure regions. These two regions may be separated by pressure nodes.

Fig. 10 shows exemplary microscope images A and B depicting hollow microrobots flowing through a 500 $\mu$m diameter Tygon® tubing before (top image A) and after (bottom image B) applying a focused ultrasonic beam using a single-lens focused ultrasound transducer. Image A of Fig. 10 may, e.g., be taken at a time t = 0 s, while image B of Fig. 10 may, e.g., be taken at t = 6 s. Using a customized Python code different regions may be defined for automized particle concentrations measurements, i.e., concentrations of hollow microrobots. Three regions of interest (ROI) are defined for characterizing the trapping efficiency. The left dashed line L1 indicates a cross section of the tubing before the focused ultrasound focal area. The dashed box $A_{trap}$ indicates an actual trapping region, i.e., a region with a pressure maximum generated within the focal area of the focused ultrasound beam, while the right dashed line L2 indicates an after trapping region in flow direction. For the images shown in Fig. 10, a flow speed of 25.47 mm/s and a focused ultrasound input voltage of 200 mVpp are applied.

To achieve efficient acoustic trapping, the concentration of hollow microrobots 110 detected inside the trapping area $A_{trap}$ should increase significantly after ultrasound exposure. The increased hollow microrobot 110 concentration is visible in bottom image B of Fig. 10, where an aggregate 116 of hollow microrobots 110 has formed after 6 seconds, indicated by a bright white spot.

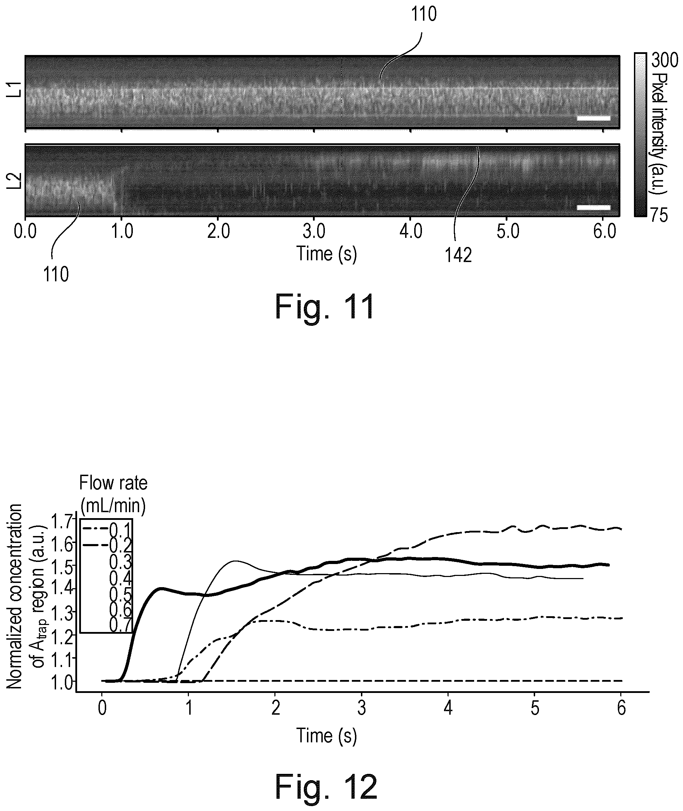

Fig. 11 shows exemplary time history images of the pixel intensity of the ROI L1 (top image A) and ROI L2 (bottom image B) cross section, as defined in Fig. 10, before and after applying the focused ultrasound. For the images shown in Fig. 11, a flow speed of 25.47 mm/s and a focused ultrasound input voltage of 200 mVpp are applied.

Upon activation of the focused ultrasound beam at around 0.9 seconds, the hollow microrobots 110 after the trapping region $A_{trap}$, i.e., at the L2 cross section, disappear from the fluid flow and move to the pipe or tubing wall 142, while the continuous stream of hollow microrobots 110 keeps running with the fluid flow as shown in the time-history plot at the L1 cross section. After a certain amount of hollow microrobots 110 has been trapped at $A_{trap}$ and an aggregate 116 has been formed, some hollow microrobots 110 leak from the trapping area $A_{trap}$ and moved to the tubing wall 142 boundary after around 3 seconds. The wall-marginated hollow microrobots 110 collecting after a few seconds are indicated by the bright pixels at the wall 142 boundary in L2 time-history plot (bottom image B).

Fig. 12 shows exemplary normalized concentrations of hollow microrobots within the trapping region $A_{trap}$ over time for flow rates reaching from 0.1 to 0.7 ml/min corresponding to flow speeds of 8.5 mm/s to 59.4 mm/s. Depicted is a sum pixel intensity of the $A_{trap}$ ROI for different flow speeds from 8.5 mm/s to 59.4 mm/s to characterize the dynamics of acoustic trapping of hollow microrobots within the trapping region $A_{trap}$.

A larger acoustic pressure in the focal area may result in a higher acoustic energy density $E_{ac}$, thus increasing the acoustic trapping force acting on the hollow microrobots. This may result in an improved trapping performance. Contrary, an increased flow rate results in a larger drag force on the hollow microrobots. The closer the drag force magnitude gets to the acoustic trapping force magnitude, the less effective the acoustic trapping may become. Furthermore, larger concentration of hollow microrobots may improve trapping performance due to particle-particle interactions $F_{pp}$, i.e., interactions between hollow microrobots, e.g., the secondary Bjerknes force and electrostatic interactions. Finally, an acoustic streaming induced drag force on the hollow microrobots $F_{AS}$ may influence the trapping performance. When statically trapping hollow microrobots, a streaming of hollow microrobots may be detected. Thus, an overall acoustic trapping force $F_{AT}$ may be described by the sum of the following forces:

$$F_{AT} = F_R + F_{PP} + F_{AS}$$

It is worth noting that using focused ultrasound for trapping a cluster of microrobots under physiological flow conditions is advantageous compared to other prevalent actuation method a such as magnetic fields. Currently, most microrobotics system rely on magnetic actuation and control. It is often favored over other control mechanisms such as optical, electrical, or chemical, e.g., due to its biocompatibility and deep penetration depth. Still, magnetic systems show limitations in terms of heating issues for electromagnets, bulkiness, and poor scalability on the micron-scale. Especially the latter presents as a major challenge in applications, where high forces, e.g., by fluids, materials or tissues need to be overcome. These challenges of state-of-the-art magnetic actuation devices may be overcome by an acoustic trapping assembly, as described herein, while maintaining their advantages. The acoustic trapping assembly, e.g., does not require continuous cooling like electromagnetic systems do. Additionally, the use of ultrasound-responsive hollow microrobots may eliminate the need for magnetic coating, avoiding potential complications arising from such a magnetic coating.

The concentrations of the trapped hollow microrobots shown in Fig. 12 are normalized to those before the focused ultrasound application. Directly after the focused ultrasound exposure, the concentration may sharply increase as shown in Fig. 12 and reached a steady state. In the steady state, no more hollow microrobots were trapped and the local concentration of hollow microrobots stayed constant. Also, the faster the fluid flow rate, the faster a rise of concentration occurs, which may be attributed to the faster rate of incoming hollow microrobots that are trapped within the focal area of the focused ultrasound wave. Since these observations are merely based on two-dimensional (2D) images, the calculations may underestimate the actual trapping performance that occurs in three-dimensional (3D) space. However, the 2D intensity estimations shown in Fig. 12 may illustrate the trend of acoustic trapping behavior of hollow microrobots under different fluid flows.

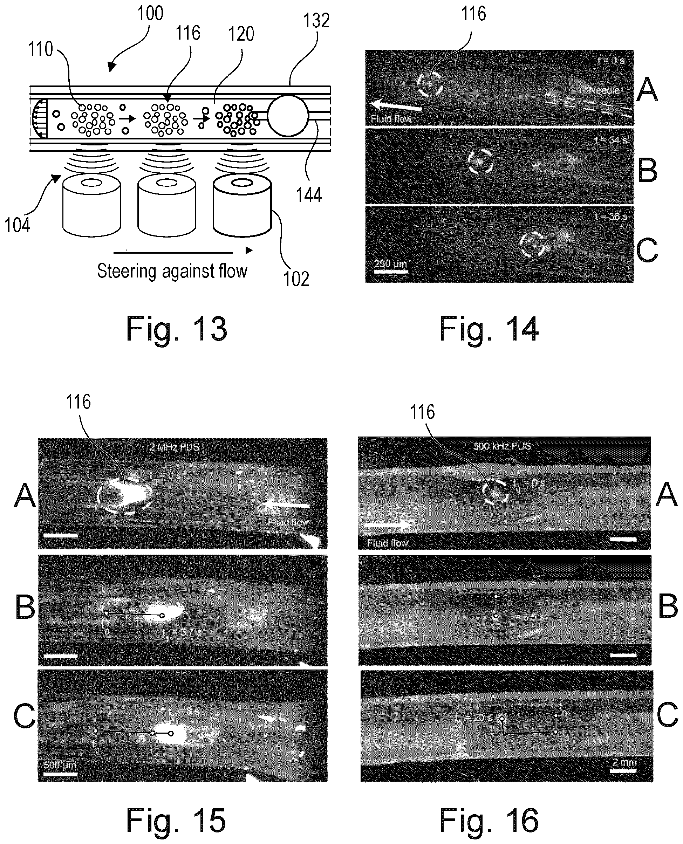

Fig. 13 shows an exemplary control and retrieval of hollow microrobots 110 against a fluid flow. Depicted is a schematic representation of a hollow microrobots 110 retrieval using acoustic manipulation enabled by a single-lensed focused ultrasound transducer 102 against the fluid flow. The single-lensed focused ultrasound transducer 102 is steered against a direction of flow navigating the aggregation 116 of hollow microrobots 110 towards an extraction device 144, e.g., a catheter, configured for extracting the hollow microrobots 110 from the tubing 132. Applications at small scales, such as in microfluidics or microbiology, need precise control and actuation of the hollow microrobots 110. The same is true, e.g., for delicate scenarios as found in medical applications, where mislead microrobots, e.g., used as drug carriers, may cause severe side effects such as occlusions. Furthermore, a retrieval of the microrobots, like in the above-mentioned fields of application, may be needed to analyze matter attached to the particles such as for antibody detection, e.g., to validate vaccine effectiveness. Also, for drug delivery scenarios such retrieval strategies, e.g., assisted using a catheter may be beneficial, as illustrated in Fig. 13. A major concern of medical products is their toxicity. The toxicity of a medical product depends inter alia on the application duration. Hence, during a registration process, e.g., of the U.S. Food and Drug Administration (FDA) and European Medicines Agency (EMA), for medical devices, higher standards are demanded for biomedical devices staying inside the body for mid- or long term. Consequently, a reliable retrieval strategy may help to improve biocompatibility of hollow microrobots used as particle-based drug carrier systems and to shorten future registration processes as medical devices.

Fig. 14 shows time laps illustrating a retrieval of hollow microrobots 110 immersed into a 4.76 mm diameter Tygon® tubing 132 against a flow speed of 0.93 mm/s. A sequence of three image A to C is shown with the first image A showing the hollow microrobots 110 at a time t = 0 s. The second image B shows the hollow microrobots 110 at a time t = 34 s. The third image C shows the hollow microrobots 110 at a time t = 36 s. The hollow microrobots 110 are trapped using a 500 kHz single-lens focused ultrasound transducer. The hollow microrobots 110 are moved against the fluid flow to an injection side using a relative movement between the tubing 132 and the transducers. For injection of the hollow microrobots 110 into the tubing, e.g., a 28G needle may be used. Afterwards, the hollow microrobots 110 are trapped acoustically and moved back to the injection side against the fluid flow. There, the hollow microrobots 110 may be retrieved, i.e., extracted, with the same needle as used for the injection.

Fig. 15 shows an exemplary movement of acoustically trapped hollow microrobots 110 inside a 500 μm diameter tubing 132. Here, 100 μL of hollow microrobots 110 with a concentration of 5 mg/mL are injected into the tubing 132 using a 28 G needle. After injection, the hollow microrobots 110 trapped using a 2 MHz single-lens focused ultrasound transducer and moved inside the tubing 132. The hollow microrobots 110 are imaged using a high-speed camera mounted to a light microscope. They may, e.g., be imaged at $t_0 = 0s$ (image A), at $t_1 = 3.7s$ (image B), and at $t_2 = 8s$ (image C). The applied flow speed is 25.46 mm/s.

This illustrates the actuation and control of the hollow microrobots 110 in confined spaces. For this experiment, the 500 $\mu$m Tygon® tubing 132 is mounted to a manual x-y-z stage. This x-y-z stage allowed for a relative movement between the tubing 132 and the transducer focus area, i.e., single-lens focused ultrasonic transducer. The applied ultrasound frequency is, e.g., 2 MHz. The hollow microrobots may be immersed in deionized (DI) water and pumped into the tubing 132 using a syringe pump, which may also enable the flow control. The hollow microrobots 110 may be moved inside the tubing 132 along a y-axis as shown in Fig. 15. A movement in x-direction may not be possible in the illustrated case as the trapped aggregate of hollow microrobots 110 may fully fill a cross-section of the tubing 132. The trapping was tested for flow speeds between 8.5 mm/s and 59.4 mm/s. The microrobots could be moved upstream at flow speeds of up to 42.4 mm/s.

Fig. 16 shows an exemplary 2D movement of trapped hollow microrobots 110 using a 500 kHz single-lens focused ultrasound transducer. Using a manual x-y-z stage, the hollow microrobots 110 are moved inside the tubing in 2D. The applied flow speed is 4.67 mm/s. For all images of Fig. 16, i.e., A to C, the trajectories of the hollow microrobots 110 as well as the flow direction are marked. They may, e.g., be imaged at $t_0 = 0s$ (image A), at $t_1 = 3.5s$ (image B), and at $t_2 = 20s$ (image C).

To demonstrate control in 2D, a 4.76 mm diameter Tygon® tubing is mounted to the x-y-z stage. Using the x-y-z stage, trapped hollow microrobots 110 can be successfully moved around in the x-y plane. The trapped hollow microrobots 110 may, e.g., be moved upstream at flow speeds of up to, e.g., 4.6 mm/s. For higher flow speeds, a higher acoustic pressure may be required. The single-lens focused ultrasound transducer used in the experiments illustrated in Fig. 16 applies a pressure of approximately 500 kPa to 600 kPa in the focal area. These flows of up to, e.g., 4.6 mm/s, may be similar to those flows occurring in human veins, enabling a potential use of this acoustic trapping assembly for vascular applications. Also, for microfluidic applications in biology, pharmacology, medicine, or chemistry, tens of micrometers up to few centimeters per second flows may expected. Thus, Fig. 16 verifies the potential of this approach for such applications.

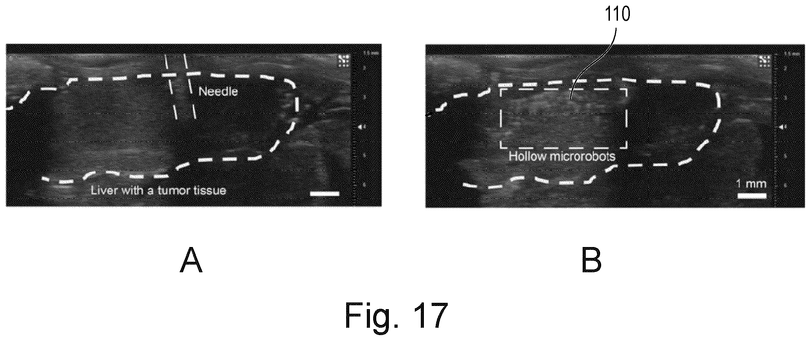

Fig. 17 shows exemplary ultrasonographic images of a needle positioning (left image A) and a subsequent hollow microrobots 110 injection (right image B) into a portal vein of an ex-vivo liver 150 of a mouse with multiple liver metastasis. The liver area, i.e., a liver contour, is marked by the dashed line. Imaging is performed in B-mode with a frequency of 40 MHz.

The hollow microrobots 110 are injected into the ex vivo mouse liver with tumor tissue to demonstrate their biomedical imaging capability in real clinical scenarios.

For this purpose, the hollow microrobots 110 are injected into the portal vein of a dead mice with liver metastases. Due to their hyperechoic appearance under ultrasound imaging, the distribution of the hollow microrobots 110 in the liver with multiple tumors is observable. In addition to their acoustic actuation capabilities, the contrast-enhancing behavior of the hollow microrobots 110 in ultrasonography makes them promising candidates for various clinical drug delivery applications, e.g., in oncology.

For the ultrasonographic examination of the hollow microrobots 110 on ex vivo liver tissues of mice with liver metastases, the hollow microrobots 110 suspension may be injected into the portal vein of the liver, e.g., with the help of a 26G needle connected to 1 ml BD Safety-Lok disposable syringe. During the injection, the localization of the needle and the hollow microrobots 110 may be observed with a high-resolution ultrasound imaging system, e.g., Vevo 3100 (FUJIFILM VisualSonics Inc., Toronto, ON, Canada). The whole procedure of locating the needle into the tissue and the followed hollow microrobots suspension injection may be imaged, e.g., with an MX Series transducer (MX550D) at the frequency of 40 MHz using B-Mode by using Vevo 3000 software.

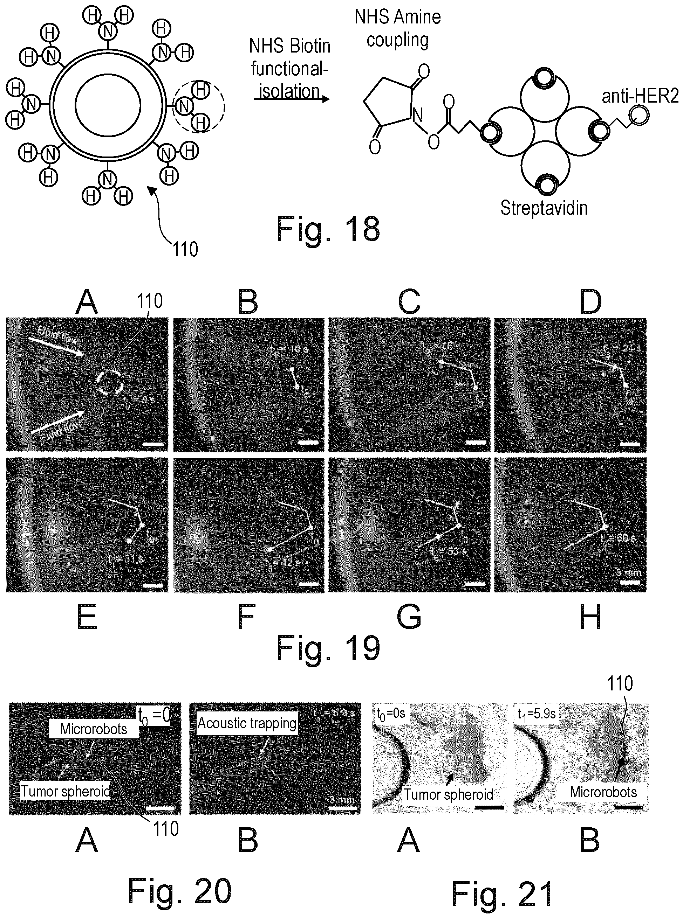

Fig. 18 shows a schematic representation of a functionalization method for binding an anti-HER2 antibody to a hollow borosilicate microrobot 110. First NH2 groups are added to the hollow microrobots 110 using (3-Aminopropyl)triethoxysilane (APTES) modification. Subsequently NHS-Biotin and Streptavidin are bound to the hollow microrobot 110. Finally, a biotinylated anti-HER2 is bound to the hollow microrobot 110 using streptavidin-biotin interaction.

To demonstrate the potential of acoustic trapping-based hollow microrobot manipulation for active targeting, the hollow borosilicate microrobots 110 have been conjugated with HER-2 antibodies. HER-2 is over-expressed by many breast cancer cells and hence a biomarker used for breast cancer targeting. Identifying or separating specific cell types or matter is a commonly used practice in cell and medical research. This is mainly done in microfluidic chips with a rectangular flow area instead of a circular as present in the tubings shown before. Consequently, the suitability of the trapping of hollow microrobots 110 using focused ultrasound waves may also be used in this scenario.

Fig. 19 shows an exemplary movement of hollow microrobots 110 inside a bifurcated microfluidic channel chip intended for cell cultures and active cell tagging. Here a fluid flow of 0.75 mL/min is applied. The flow direction is indicated by the arrows. From the starting position at time $t_0$ (image A) the hollow microrobots 110 are moved in the top bifurcation against the flow. Afterwards the hollow microrobots 110 are moved back to the starting position in direction of the flow. Finally, the hollow microrobots 110 are moved to the bottom bifurcation and back to the starting point. For acoustic trapping a 2 MHz single-lens focused ultrasound transducer is used. The current trapping region is marked in each image by a dotted circle.

In image A of Fig. 19, the position of the hollow microrobots 110 at $t_0 = 0$ s is shown, in image B the position at $t_1 = 10$ s, in image C the position at $t_2 = 16$ s, in image D the position at $t_3 = 24$ s, in image E the position at $t_4 = 31$ s, in image F the position at $t_5 = 42$ s, in image G the position at $t_6 = 53$ s, and in image H the position at $t_7 = 60$ s.

For applications of the acoustic trapping assemble, besides the movement of trapped hollow microrobots 110 against fluid flows, also their controlled movement into bifurcations may be beneficial. Hence, bifurcated microchannels with a width of 3 mm and a height of 0.4 mm are used with differing flow rates to test the precise movement of hollow microrobots 110 into both branches of the microchannel. To allow movement in the x-y-z direction, the microchannel may be mounted to a manual x-y-z stage. After an injection of the hollow microrobots 110 into a tubing connected to the microchannel, they are transported into the field of view. When the hollow microrobots 110 are flushed into the microchannel, they are trapped using the 2 MHz single-lens focused ultrasound transducer. The controlled movement of hollow microrobots 110 in and out of the bifurcation may be demonstrated under varying flows of, e.g., 0.25 mL/min, 0.5 mL/min, 0.75 mL/min and 1 mL/min. Considering the channel dimensions, this corresponds to flow speeds of, e.g., 34.7 mm/s up to 139 mm/s. Successful trapping and movement of hollow microrobots 110 may be demonstrated up to a flow rate of 0.75 mL/min, corresponding to a flow speed of 104.2 mm/s as shown in Fig. 19. As previously described, these flow speeds suffice the requirements for microfluidic applications. The large difference of flow velocities in cylindrical channels and rectangular channels are attributed to the geometry of the channels. Due to the rectangular shape more, hollow microrobots 110 may be present at the channel's walls where they experience significantly less flow than in the middle of the channel. This may allow for movement against even high flow speeds.

The setup used may, e.g., consist of a single-lens focused ultrasound transducer with a central frequency of 2 MHz (SU-101, Sonic Concepts, U.S.A) embedded, e.g., into a customized glass tank with dimensions of 30 x 30 x 20 cm. The transducer may be fixed to the bottom of the tank using double-sided adhesive and connected to an amplifier. A function generator (AFG 1022 Tektronix GmbH, Germany) may be attached to the amplifier and generate the input for the amplifier including input voltage and frequency. To measure the output signal of the amplifier also an oscilloscope (MDO 4042C Tektronix GmbH, Germany) may be connected to the amplifier. To determine the ability of the focused ultrasound trapping inside a bifurcated channel under flow, a cell culture chip may be used (μ-Slide y-shaped, ibidi, Germany). For precise positioning, the channel may be attached to a customized holder connected to an x-y-z stage. To provide flow inside the channel, tubings may be attached to the chip and a syringe pump, while being sealed using Luer locks. The residue may be collected in a reservoir. The hollow microrobots may be injected into the tubing using, e.g., a 24 G needle and a 1 ml volume syringe.

Fig. 20 shows exemplary microscope images A and B depicting a trapping and subsequent movement of hollow microrobots 110 through a microfluidic channel towards a tumor spheroid. When in close proximity to the tumor spheroid, the hollow microrobots 110 attach to the tumor spheroid and escape the acoustic trap at t=5.93 sec. For the experiments a concentration of $1 \times 10^7$ cells per ml may be seeded into a commercially available bifurcated microfluidic chip. The cells may be incubated inside the upside-down chip for 2h. Hence, the cells attached to the top of the chip ensure contact between the breast cancer cells and the buoyant hollow microrobots 110 after injection into the chip. After injection into the chip, the hollow microrobots 110 are acoustically trapped using the 2 MHz single-lens focused ultrasound transducer and moved towards the cancer spheroid, as demonstrated in Fig. 20. The microscope images shown in Fig. 20 are taken at time $t_0 = 0$s (left image A) and $t_1 = 5.9$s (right image B).

Fig. 21 shows exemplary light microscope images A and B of SKBR3 cells (immortalized human breast cancer cell line) embedded into a cell culture chip. The left image A is taken at $t_0 = 0$s. The right image B is taken at $t_1 = 5.9$s after injection and FUS-enabled movement of anti-HER2 functionalized hollow microrobots 110 to the cancer spheroid. The hollow microrobots 110 are bound to the cells by an antibody-receptor interaction. Close to the cancer spheroid, the hollow microrobots 110 leave the acoustic trap and attach to the front side of the cancer spheroid as shown in image B of Fig. 21. Such an active targeting may have various applications, e.g., in the field of cell- and microbiology as well as for biomedical research. Due to the small size of the transducer probe, this technology may easily be incorporated into a bench top research setup, especially when using a probe with a shorter focal distance.

For a functionalization of the hollow borosilicate microrobots, the hollow microrobots may first be functionalized inside an Eppendorf tube using, e.g., (3-Aminopropyl)triethoxysilane 99% (APTES) diluted to a concentration of 5% inside Ethanol. The Eppendorf may be sealed using, e.g., Parafilm and aluminum foil, and placed on a shaker, e.g., at 2400 rpm overnight. Afterward the hollow microrobots may be placed on a heater, e.g., at 65°C for 1h. After washing the hollow microrobots, e.g., four times using Dimethyl Sulf oxide (DMSO), 5 mg/ml of N-Hydroxysuccinidobiotin (NHS Biotin) may be added to the hollow microrobots. The hollow microrobots may be sealed again and put into the shaker, e.g., at 2400 rpm for 3h. Furthermore, the hollow microrobots may be washed, e.g., four times using phosphate-buffered saline solution (PBS), and, e.g., 20% (200 μl/ml) of Streptavidin may be added. The hollow microrobots may be placed in the shaker, e.g., for 1h at 2400 rpm. Subsequently the hollow microrobots may be washed with PBS, e.g., four times, and 50 µl of biotinylated anti-human HER-2 may be added to the hollow microrobots.

To illustrate the ability of HER-2 functionalized hollow borosilicate microrobots to tag, e.g., breast cancer cells (SKBR3) the same setup as described for the acoustic trapping inside a bifurcated channel may be used. The chip (µ-Slide y-shaped, ibidi, Germany) may be first rinsed with Ethanol and afterwards with sterile PBS under a fume hood. To further decrease a risk of contaminations, the chip may be placed under UV light, e.g., for 1h. Thereby, the presence of a PBS meniscus may be ensured at the channel opening to avoid evaporation. Further, the channel may be rinsed, e.g., with diluted fibronectin (dilution 1:10, 50 µl fibronectin + 450 µl PBS) and placed upside-down inside an incubator, e.g., at 37°C for 1h. The channel may be flushed with the cell culture medium to remove unbound fibronectin. Then, SKBR3 cells may be seeded into the chip using a density of 500.000 cells per ml. The chip may be placed upside down into a petri-dish and placed inside the incubator, e.g., for 2h. The orientation of the chip may be relevant to ensure that the hollow microrobots also get into contact with the cells during passive injection. Since the hollow microrobots are buoyant, they may move to the top of the channel. Consequently, cells may be seeded to the top part of the channel. Finally, the channels may be flushed with PBS, and microscope images may be taken to ensure the attachment of cells.

While the invention has been illustrated and described in detail in the drawings and foregoing description, such illustration and description are to be considered illustrative or exemplary and not restrictive; the invention is not limited to the disclosed embodiments.

**REFERENCE SIGNS LIST**

| | |
|---|---|
| 100 | acoustic trapping assembly |
| 102 | focused ultrasound transducer |
| 104 | focused ultrasound beam |
| 106 | focal area |
| 110 | hollow microrobots |
| 112 | solid shell |
| 114 | core |
| 116 | aggregate |
| 120 | medium |
| 122 | pipe |
| 130 | setup |
| 132 | tubing |
| 134 | tank |
| 136 | x-y-z stage |
| 138 | syringe pump |
| 140 | residue reservoir |
| 142 | pipe wall |
| 144 | extraction device |

| | |
|---|---|
| $\alpha$ | ratio of inner radius to outer radius |
| $r_i$ | inner radius |
| $r_o$ | outer radius |
| $\phi$ | acoustophoretic contrast factor |
| $A_{trap}$ | trapping area |
| $F_B$ | buoyancy force |
| $F_G$ | gravitational force |
| $F_T$ | trapping force |
| $F_D$ | drag force |

**Claims**

1. An acoustic trapping assembly (100) comprising a focused ultrasound transducer (102) and a plurality of hollow microrobots (110), the focused ultrasound transducer (102) being configured for generating an acoustic pressure maximum within a focal area (106) of a focused ultrasound beam (104) emitted by the focused ultrasound transducer (102) with the plurality of hollow microrobots (110) being acoustically trapped within the acoustic pressure maximum under fluid flow in a fluidic medium (120), the hollow microrobots (110) comprising solid shells (112) filled with gas.

2. The acoustic trapping assembly (100) of claim 1, the focused ultrasound transducer (102) being a single-lens focused ultrasound transducer.

3. The acoustic trapping assembly (100) of any of the preceding claims, the acoustically trapped plurality of hollow microrobots (110) forming an aggregate (116).

4. The acoustic trapping assembly (100) of any of the preceding claims, the hollow microrobots (110) being polydisperse or monodisperse regarding one or more of the following: form, size, shell thickness.

5. The acoustic trapping assembly (100) of any of the preceding claims, the hollow microrobots (110) having spherical forms.

6. The acoustic trapping assembly (100) of any of the preceding claims, the hollow microrobots (110) having a size within a range from 2.5 µm to 17.5 µm, preferable from 8 µm to 12 µm.

7. The acoustic trapping assembly (100) of any of the preceding claims, a ratio ($\alpha$) of an inner radius ($r_i$) to an outer radius ($r_o$) of the hollow microrobots (110) being at least 0.90, preferably at least 0.95, and more preferably at least 0.98.

8. The acoustic trapping assembly (100) of any of the preceding claims, the solid shells (112) of the hollow microrobots (110) having a thickness within a range from 100 nm to 1 µm , preferably from 250 nm to 750 nm, and more preferably from 400 nm to 600 nm.

9. The acoustic trapping assembly (100) of any of the preceding claims, the solid shells (112) of the hollow microrobots (110) being gas-tight.

10. The acoustic trapping assembly (100) of any of the preceding claims, the solid shells (112) of the hollow microrobots (110) comprising at least one layer made from borosillicate.

11. The acoustic trapping assembly (100) of any of the preceding claims, the solid shells (112) of the hollow microrobots (110) comprising a coating.

12. The trapping assembly (100) of claim 11, the coating of the solid shells (112) of the hollow microrobots (110) comprising a reagent.

13. The acoustic trapping assembly (100) of any of the preceding claims, the focused ultrasound beam (104) emitted by the focused ultrasound transducer (102) having a frequency within a range from 250 kHz to 5 MHz, preferably within a range from 500 kHz to 3.5 MHz.

Fig. 1

Fig. 2

Fig. 3

Fig. 4

Fig. 5

Fig. 6

EP 4 487 801 A1

Acoustic pressure (MPa)

Fig. 8

Acoustic pressure (kPa)

Fig. 7

Fig. 9

Fig. 10

Fig. 11

Fig. 12

Fig. 13

Fig. 14

Fig. 15

Fig. 16

110

Hollow microrobots

1 mm

B

Needle

Liver with a tumor tissue

A

Fig. 17

NHS Amine
coupling

NHS Biotin
functional-
isolation

Streptavidin

anti-HER2

Fig. 18

110

A 110    B    C    D

E    F    G    H

Fig. 19

A 110    B

Fig. 20

110

A    B

Fig. 21

Europäisches
Patentamt

European
Patent Office

Office européen
des brevets

# EUROPEAN SEARCH REPORT

**Application Number**

**EP 23 18 3898**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEIBACHER IVO ET AL: "Acoustophoresis of hollow and core-shell particles in two-dimensional resonance modes", MICROFLUIDICS AND NANOFLUIDICS, vol. 16, no. 3, 7 August 2013 (2013-08-07), pages 513-524, XP093105433, Berlin/Heidelberg ISSN: 1613-4982, DOI: 10.1007/s10404-013-1240-7 Retrieved from the Internet: URL:https://link.springer.com/content/pdf/10.1007/s10404-013-1240-7.pdf> | 1,3-10, 13 | INV. A61B34/00 A61K9/00 A61K9/48 A61K9/50 |
| Y | * pages 521-523; figures 8-11 * | 2,11,12 | |
| Y | LEE HAN-SOL ET AL: "Medical Microrobot - Wireless Manipulation of a Drug Delivery Carrier through an External Ultrasonic Actuation: Preliminary Results", INTERNATIONAL JOURNAL OF CONTROL, AUTOMATION AND SYSTEMS, KOREAN INSTITUTE OF ELECTRICAL ENGINEERS, SEOUL, KR, vol. 18, no. 1, 28 November 2019 (2019-11-28), pages 175-185, XP036979407, ISSN: 1598-6446, DOI: 10.1007/S12555-019-0239-6 [retrieved on 2019-11-28] * pages 180-181; figures 2a, 4 * | 2,11,12 | TECHNICAL FIELDS SEARCHED (IPC) A61B A61K |

-/--

The present search report has been drawn up for all claims

| Place of search | Date of completion of the search | Examiner |
|---|---|---|
| The Hague | 26 November 2023 | Schmidt, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 18 3898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOO JINHEE ET AL: "Collapse pressure measurement of single hollow glass microsphere using single-beam acoustic tweezer", ULTRASONICS SONOCHEMISTRY, BUTTERWORTH-HEINEMANN, GB, vol. 82, 25 November 2021 (2021-11-25), XP086938429, ISSN: 1350-4177, DOI: 10.1016/J.ULTSONCH.2021.105844 [retrieved on 2021-11-25] * pages 2-3; figure 1 * | 1,2,4,5, 8-10 | |
| X | US 2019/056302 A1 (BEREZIN MIKHAIL [US] ET AL) 21 February 2019 (2019-02-21) * paragraphs [0052], [0084], [0126], [0131]; example 1 * | 1,3-6, 9-13 | |
| | | | **TECHNICAL FIELDS SEARCHED (IPC)** |

The present search report has been drawn up for all claims

| Place of search | Date of completion of the search | Examiner |
|---|---|---|
| The Hague | 26 November 2023 | Schmidt, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 18 3898**

This annex lists the patent family members relating to the patent documents cited in the above-mentioned European search report.
The members are as contained in the European Patent Office EDP file on
The European Patent Office is in no way liable for these particulars which are merely given for the purpose of information.

**26-11-2023**

| Patent document cited in search report | Publication date | Patent family member(s) | | Publication date |
|---|---|---|---|---|
| US 2019056302 A1 | 21-02-2019 | US | 2019056302 A1 | 21-02-2019 |
| | | US | 2021239591 A1 | 05-08-2021 |

EPO FORM P0459

For more details about this annex : see Official Journal of the European Patent Office, No. 12/82